# EUROPÄISCHE PATENTANMELDUNG

(11) **EP 0 755 693 A1**
(43) Veröffentlichungstag der Anmeldung: **29.01.1997**
(21) Anmeldenummer: 95111243.2
(22) Anmeldetag: 18.07.1995
(51) Int. Cl.: A61M 25/01

(54) **Katheter-Führungsdraht**

(71) Anmelder: SCHNEIDER (EUROPE) AG, CH-8180 Bülach (CH)
(72) Erfinder: Schwager, Michael, CH-8404 Winterthur (CH)
(74) Vertreter: Schwepfinger, Karl-Heinz, Dipl.-Ing.

(57) **Zusammenfassung**

Ein Katheter-Führungsdraht weist einen Hauptabschnitt (12) mit kreisförmigem Querschnitt und einen distalen, von einer Spirale (20) umgebenen Endabschnitt (14) auf. Der distale Endabschnitt (14) hat einen an die Führungsdrahtspitze angrenzenden Bereich (18) in Form eines Keils, dessen Höhe zur Führungsdrahtspitze hin abnimmt und dessen Breite zur Führungsdrahtspitze hin zunimmt.

## Beschreibung

Die Erfindung bezieht sich auf einen Katheter-Führungsdraht mit einem Hauptabschnitt mit kreisförmigem Querschnitt und einem distalen, von einer Spirale umgebenen Endabschnitt.

Aus der EP-A-0 279 959 ist ein Katheter-Führungsdraht bekannt, dessen Endabschnitt einen sich zur Führungsdrahtspitze hin in mehreren Stufen verringernden Durchmesser hat. In der unmittelbar an der Führungsdrahtspitze liegenden Stufe ist der Führungsdraht abgeflacht und hat einen Rechteckquerschnitt bei gleichbleibender Dicke und Breite. Die stufenförmige Durchmesserverringerung zur Führungsdrahtspitze hin hat den Zweck, die Flexibilität des Drahts in diesem Bereich zu verringern. Es muß aber trotz der Durchmesserverringerung stets gewährleistet sein, daß ein am proximalen Führungsdrahtende eingebrachtes Drehmoment bis zum distalen Endbereich des Führungsdrahts übertragen wird, damit der Draht bei seiner Anwendung steuerbar bleibt. Wegen der Forderung der Drehmomentübertragung und auch aus Festigkeitsgründen kann der Durchmesser des Führungsdrahts an der Drahtspitze nicht beliebig verringert werden, so daß bei dem bekannten Führungsdraht anstelle einer Durchmesserverringerung die Abflachung des unmittelbar an die Führungsdrahtspitze angrenzenden Abschnitts angewendet wird. Durch die Abflachung wird eine sehr hohe Flexibilität erreicht, ohne daß die Formbarkeit der Spitze und die Drehmomentübertragung übermäßig beeinflußt werden. An der Übergangsstelle zwischen einer im Durchmesser verringerten Stufe und dem flach ausgebildeten Abschnitt an der Führungsdrahtspitze tritt ein Flexibilitätssprung ein, und die Flexibilität bleibt dann im gesamten flach ausgebildeten Abschnitt konstant. An dieser Übergangsstelle kann es auch zu Festigkeitsproblemen kommen.

Der Erfindung liegt die Aufgabe zugrunde, einen Katheter-Führungsdraht der eingangs geschilderten Art zu schaffen, der unter Aufrechterhaltung seiner Formbarkeit und seiner Fähigkeit zur Drehmomentübertragung ohne Festigkeitsprobleme im Bereich seiner Spitze eine sehr hohe Flexibilität aufweist.

Diese Aufgabe wird gemäß der Erfindung dadurch gelöst, daß der distale Endabschnitt einen an die Führungsdrahtspitze angrenzenden Bereich in Form eines Keils hat, dessen Höhe zur Führungsdrahtspitze hin abnimmt und dessen Breite zur Führungsdrahtspitze hin zunimmt.

Aufgrund dieser Ausgestaltung des erfindungsgemäßen Katheter-Führungsdrahts ergibt sich in dem an die Führungsspitze angrenzenden Bereich eine stetige Zunahme der Flexibilität, ohne daß die übrigen, an das Verhalten des Führungsdrahts im Endabschnitt gestellten Anforderungen beeinträchtigt werden.

Die Erfindung wird nun anhand der Zeichnung beispielshalber erläutert. Es zeigen:
- Fig. 1: eine Ansicht des Endabschnitts eines Katheter-Führungsdrahts nach der Erfindung, wobei der in Form eines Keils ausgebildete Bereich von der Seite zu erkennen ist, und
- Fig. 2: eine Ansicht des Endabschnitts des Katheter-Führungsdrahts von Fig. 1 mit einer Draufsicht auf den keilförmig ausgebildeten Bereich.

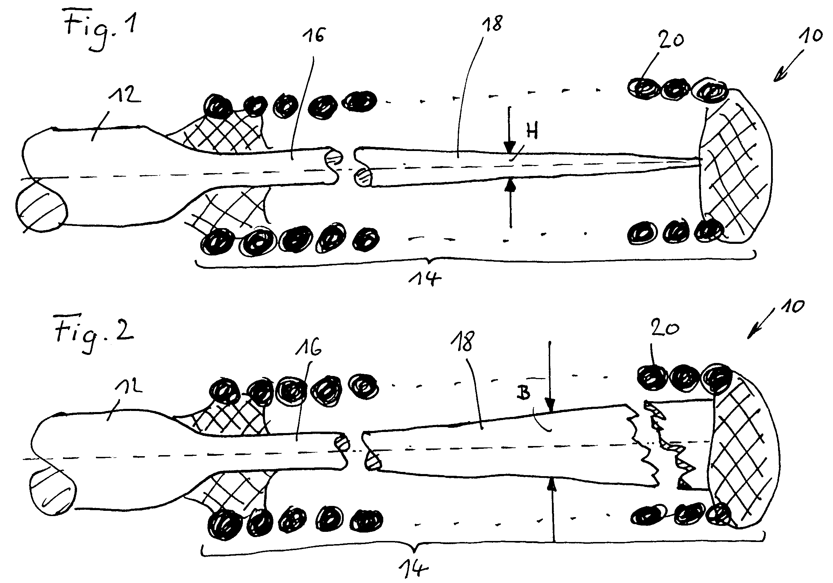

Der in Fig. 1 dargestellte Führungsdraht 10 weist einen Hauptabschnitt 12 auf, von dem nur ein kleiner Teil zu erkennen ist. Dieser Hauptabschnitt 12 hat einen kreisförmigen Querschnitt. An diesen Hauptabschnitt schließt sich ein Endabschnitt 14 an, der aus einem Bereich 16 mit reduziertem, kreisförmigem Querschnitt und einem an die Führungsdrahtspitze angrenzenden Bereich 18 in Form eines Keils besteht. Zur Führungsdrahtspitze hin nimmt die Höhe H des Keils ab, während seine Breite B zunimmt. Der gesamte Endabschnitt 14 ist von einer Spirale 20 umgeben, die unmittelbar an der Führungsdrahtspitze und im Bereich des Übergangs zwischen dem Hauptabschnitt 12 und dem Endabschnitt 14 mit dem Führungsdraht beispielsweise durch Kleben, Löten oder dergleichen fest verbunden ist.

Die Figuren 1 und 2 lassen erkennen, daß im Endabschnitt 14 ein stetiger Übergang zwischen dem Bereich 16 mit reduziertem Kreisquerschnitt und dem keilförmig ausgebildeten Bereich 18 vorhanden ist. In diesem Übergangsbereich entsprechen die Höhe und die Breite des Keils an seinem proximalen Ende somit dem Durchmesser des Bereichs 16. Der Endbereich 14 wird also zur Führungsdrahtspitze hin aufgrund der keilförmigen Ausgestaltung des Bereichs 18 immer flexibler, da die Keilhöhe H kontinuierlich abnimmt. Aufgrund des stetigen Übergangs zwischen dem Bereich 16 mit reduziertem Kreisquerschnitt und dem keilförmigen Bereich 18 tritt keine Beeinträchtigung der Festigkeit im Übergang zwischen den beiden Bereichen auf. Insbesondere besteht in diesem Übergangsbereich keine Knickanfälligkeit, und die maximale Kontrolle der Bewegung der Führungsdrahtspitze bleibt erhalten. Ein weiterer Vorteil des stetigen Übergangs ist darin zu sehen, daß beim Vorschieben des Führungsdrahts durch kurvenreiche Verengungen in Gefäßen beim Abstützen des Führungsdrahts an der Gefäßwand keine Steifigkeitsstufen mehr bemerkbar sind.

## Patentansprüche

1. Katheter-Führungsdraht mit einem Hauptabschnitt (12) mit kreisförmigem Querschnitt und einem distalen, von einer Spirale umgebenen Endabschnitt (14), dadurch gekennzeichnet, daß der distale Endabschnitt (14) einen an die Führungsdrahtspitze angrenzenden Bereich in Form eines Keils hat, dessen Höhe zur Führungsdrahtspitze hin abnimmt und dessen Breite zur Führungsdrahtspitze hin zunimmt.

2. Katheter-Führungsdraht nach Anspruch 1, dadurch gekennzeichnet, daß der Endabschnitt (14) zwischen dem Hauptabschnitt (12) und dem keilförmigen Bereich (18) mindestens einen Bereich (16) mit reduziertem Kreisquerschnitt aufweist, daß die Höhe und die Breite des Keils an seinem proximalen Ende dem Druchmesser des in proximaler Richtung angrenzenden Bereichs (16) entsprechen und daß das distale Ende des Keils die Führungsdrahtspitze ist.
